# EUROPÄISCHE PATENTSCHRIFT

(11) **EP 1 069 116 B1**
(45) Veröffentlichungstag und Bekanntmachung des Hinweises auf die Patenterteilung: **04.06.2003**
(21) Anmeldenummer: 00113133.3
(22) Anmeldetag: 29.06.2000
(51) Int. Cl.: C07D 277/04, C11B 9/00, A61K 7/46, A23L 1/226

(54) **N-(2-Mercaptoethyl)-1,3-thiazolidine und deren Verwendung als Riech- und Aromastoffe**
N-(2-Mercaptoethyl)-1,3-thiazolidines and their use as fragrances and flavourings
N-(2-Mercaptoéthyl)-1,3-thiazolidines et leur utilisation comme parfums et arômes

(30) Priorität: 12.07.1999 DE 19932495
(43) Veröffentlichungstag der Anmeldung: 17.01.2001
(73) Patentinhaber: HAARMANN & REIMER GMBH, 37601 Holzminden (DE)
(72) Erfinder: Engel, Wolfgang, 80538 München (DE); Schieberle, Peter, Prof. Dr., 85354 Freising (DE); Güntert, Matthias, Dr., Teterboro, NJ 07608 (US); Lambrecht, Stefan, Dr., 37603 Holzminden (DE)
(74) Vertreter: Eisenführ, Speiser & Partner

(56) Entgegenhaltungen:
- FR-A- 1 428 902
- US-A- 3 980 089
- DATABASE CAOLD [Online] CHEMICAL ABSTRACTS SERVICE, COLUMBUS, OHIO, US; Accession Number CA55 : 796i, XP002149362 -& CHEMICAL ABSTRACTS, vol. 56, no. 1, 9. Januar 1961 (1961-01-09) Columbus, Ohio, US; abstract no. 796i, TANK L I : "Protective effect of aminothiols and of their derivatives against ionizing radiation" XP002149360 & TIOLOVYE SOEDINEN. V MED., UKRAIN. NAUCH.-ISSLEDOVATEL. SANIT.-KHIM. INST., TRUDY NAUCH. KONF., KIEV, 1957, Seiten 260-269,
- CHEMICAL ABSTRACTS, vol. 104, no. 13, 31. März 1986 (1986-03-31) Columbus, Ohio, US; abstract no. 109619a, Seite 726; XP002149361 & JP 60 149592 A (HASEGAWA, T., CO., LTD.) 7. August 1985 (1985-08-07)

## Beschreibung

Die Erfindung betrifft N-(2-Mercaptoethyl)-1,3-thiazolidine, Verfahren zu deren Herstellung und deren Verwendung als aromatisierender Stoff in Nahrungs- und Genussmitteln sowie zum Versprühen von Lebensmitteldüften und zur Raumluftaromatisierung.

In der Aromenindustrie besteht nach wie vor ein starker Bedarf an Stoffen, die Nahrungs- und Genussmitteln einen olfaktorischen Eindruck vermitteln, wie er bei der thermischen Behandlung während des Koch-, Back- und Röstprozesses von Lebensmitteln entsteht. Die hierbei entstehenden aromatisierenden Verbindungen weisen vor allem röstige Noten auf. Insbesondere diese Verbindungen sind aber bislang nur wenig verfügbar. Neben dem Geschmack, der größtenteils in Wirklichkeit retronasal als Geruch wahrgenommen wird, spielt heute der orthonasal wahrgenommene Geruch eine große Rolle. Deshalb sind auch Aromaverbindungen wichtig, die einem Lebens- und Genussmittel einen entsprechend starken und typischen Geruch verleihen.

Die wichtigste Reaktion, die bei der thermischen Behandlung von Lebensmitteln verläuft, ist die Reaktion zwischen reduzierenden Zuckern und Aminosäuren, die sogenannte Maillard-Reaktion. Es entstehen während dieser Maillard-Reaktion insbesondere Aromastoffe aus der chemischen Substanzklasse der Heterocyclen. Diese Verbindungen enthalten ein oder mehrere Heteroatome, verschiedene Seitenketten und sind aromatisch oder teilweise hydriert (P.A. Finot, H.U. Aeschbacher, R.F. Hurrell, R. Liardon, *The Maillard Reaction in Food Processing, Human Nutrition and Physiology,* Birkhäuser Verlag, Basel, 1990).

Trudy Nauch. Konf., Kiev 1957, 260-9 beschreibt den positiven Effekt von Aminothiolen und deren Derivaten gegen ionisierende Strahlung und nennt als Beispiel 2,2-Dimethyl-3-thiazolidinethanthiol.

JP-A 60 149 592 offenbart die Herstellung von 2-Alkylperhydrothiazolo[4,4-b]thiazolen und ihre Verwendung als Aromastoffe.

Als neue Substanzklasse von Riech- und Aromastoffen wurden N-(2-Mercaptoethyl)-1 ,3-thiazolidine und deren Disulfide der Formel 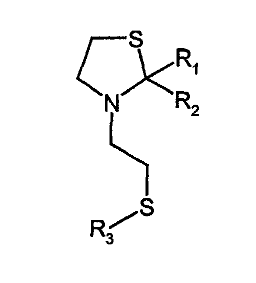 gefunden, in denen R₁, R₂ und R₃ gleich oder verschieden sind und R₁ Wasserstoff, Methyl, Ethyl, Propyl, iso Propyl bedeutet, R₂ Wasserstoff, Acetyl, Propionyl, Isobutyryl, Methyl, Ethyl, Propyl, iso Propyl und R₃ Wasserstoff oder 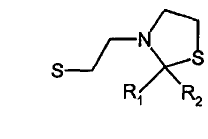 bedeutet.

Gegenstand der Erfindung sind daher die oben genannten N-Mercaptoethyl-1,3-thiazolidine und deren Disulfide mit Ausnahme von 2,2-Dimethyl-3-thiazolidinethanthiol.

Substituierte N-Mercaptoethyl-1,3-thiazolidine und deren Disulfide sind Riech- und Aromastoffe.

Die erfindungsgemäßen substituierten N-Mercaptoethyl- 1 ,3-thiazolidine und deren Disulfide, insbesondere N-(2-Mercaptoethyl)-1,3-thiazolidin, haben überraschenderweise sehr niedrige Schwellenwerte. Beispielsweise hat das erfindungsgemäße N-(2-Mercaptoethyl)-1,3-thiazolidin mit 0,005 ng/l in Luft einen überraschend niedrigen Schwellenwert. Dies ermöglicht die Verwendung als aromatisierenden Bestandteil in kleinsten Konzentrationen. Außerdem verleiht N-(2-Mercaptoethyl)-1,3-thiazolidin Lebensmitteln ein besonders starkes und typisches, röstiges Aroma.

Über die Substanzklasse der Thiazolidine ist im Vergleich zu den Thiazolinen, bei denen insbesondere das 2-Acetyl-2-thiazolin ein bekannter Aromastoff ist (J. Kerler, J.G.M. van der Veen, H. Weenan, *Food Rev. Int.,* 1997, 13, 553-575), als Aromastoffe bisher wenig bekannt. Zwar sind in der wissenschaftlichen Literatur als Reaktionsprodukte von Zuckern und Aminosäuren auch Thiazolidine beschrieben, diese zeichnen sich allerdings nicht durch entsprechende aromatisierende Eigenschaften aus und treten deshalb als industriell verwendete Aromastoffe auch nicht in Erscheinung.

Überraschenderweise entfaltet N-(2-Mercaptoethyl)-1 ,3-thiazolidin in wässrigen Lösungen praktisch keinerlei retronasale Aromawirkung, das heißt, er wird geschmacklich beim Verkosten kaum als aromagebend erkannt. Allerdings hat es eine sehr starke orthonasale Wirkung und entwickelt eine intensiv röstige Geruchsnote. Damit hat der erfindungsgemäße Aromastoff N-(2-Mercaptoethyl)-1,3-thiazolidin zwar ähnliche sensorische Eigenschaften wie Furfürylthiol, ist aber deutlich geruchsstärker und hat einen deutlich tieferen Geruchsschwellenwert. Dieser wurde zu 0,005 ng/l in Luft bestimmt. Die Verbindung gehört damit zu den aromastärksten Verbindungen, die in der Aromachemie bekannt sind

N-(2-Mercaptoethyl)-1,3-thiazolidin wurde als thermisches Reaktionsprodukt von Cysteamin mit Fructose identifiziert (Beispiel 1). Seine strukturelle Herkunft ist außerdem nicht einfach zu erklären. Vermutlich tragen Cysteamin und Formaldehvd zu seiner thermischen Bildung bei. Die Identifizierung gelang durch Auftrennung des Extraktes aus dem Reaktionsgemisch mittels Gas-Chromatogaphie und anschließender massenspektrometrischer Untersuchung. N-(2-Mercaptoethyl)-1,3-thiazolidin konnte durch Vergleich mit den analytischen Daten einer authentischen Probe eindeutig identifiziert werden.

Darüberhinaus wurden auch systematische Versuche mit einer chromatographischen Technik durchgeführt, die als Gaschromatographie-Olfaktometrie (GC-O) bezeichnet wird. Dabei werden die während des Chromatographievorgangs aufgetrennten Verbindungen am Ende der Trennkapillare mit der Nase einzeln abgerochen. Mit Hilfe dieser Techniken konnten die geruchlichen und geschmacklichen Qualitäten von N-(2-Mercaptoethyl)-1,3-thiazolidin vorbewertet werden.

Die Struktur wurde durch Vergleich mit synthetisch hergestelltem N-(2-Mercaptoethyl)-1,3-thiazolidin bewiesen. N-(2-Mercaptoethyl)- 1 ,3-thiazolidin kann ausgehend von Thiazolidin hergestellt werden. Thiazolidin wird mit Ethylensulfid versetzt und unter Inertgasatmosphäre 24 Stunden auf 80°C erhitzt. Man erhält nach Reinigung N-(2-Mercaptoethyl)-1,3-thiazolidin (Beispiel 2).

Die erfindungsgemäße Verbindung N-(2-Mercaptoethyl)-1,3-thiazolidin eignet sich wegen ihres herausragenden organoleptischen Charakters vorzüglich als Aromastoff für den Einsatz in Aromakompositionen. Besonders überraschend ist, dass das N-(2-Mercaptoethyl)-1,3-thiazolidin den betreffenden Kompositionen eine sehr intensive röstige Note in ausgesprochen niedrigen Konzentrationen verleiht. Dabei wird das Aroma besonders geruchlich (orthonasal) wahrgenommen.

In gebrauchsfertigen Aromen liegt die eingesetzte Menge der erfindungsgemäßen Verbindung vorzugsweise zwischen 0,00005 und 1 Gew.-%, insbesondere zwischen 0,0001 und 0,5 Gew.-%, bezogen auf die gesamte Komposition. Derartige Aroma-Kompositionen können im gesamten Nahrungs- und Genussmittelbereich verwendet werden. Insbesondere sind sie geeignet für die Aromatisierung von Snacks, Suppen, Saucen, Fertiggerichten, Fettmassen, Backwaren, Joghurt, Speiseeis und Süßwaren. Die Dosierung derartiger Aroma-Kompositionen liegt vorzugsweise bei 0,005 bis 2 Gew.-%, insbesondere zwischen und 0,01 und 1 Gew.-% bezogen auf das fertige Lebens- oder Genussmittel.

Die gebrauchsfertigen Aromen können in flüssiger oder sprühgetrockneter oder verkapselter Form eingesetzet werden. Während sie in flüssiger Form in einem praxisüblichen Lösungsmittel wie Ethanol, Propylenglycol, Pflanzenöltriglyceriden oder Triacetin eingesetzt werden, gewinnt man die trockenen Aromen durch Sprühtrocknung oder durch Verkapselung nach einem in der Aromenindustrie üblichen Verfahren. Dies sind insbesondere die Extrusion und die Sprühgranulation.

Durch die starke geruchliche Wirkung eignet sich die erfindungsgemäße Verbindung N-(2-Mercaptoethyl)-1,3-thiazolidin insbesondere auch zur Aromatisierung von Verpackungen von Lebens- und Genussmitteln wie auch für andere Einsatzzwecke (z.B. das Versprühen von Lebensmitteldüften und zur Raumluftaromatisierung).

### Beispiele

### Beispiel 1: Reaktion zwischen Cysteamin und Fructose

3,3 mmol Cysteamin und 10 mmol Fructose werden in einem Phophatpuffer bei pH 7 in 20 Minuten auf 145°C erhitzt. Die Aromastoffe werden durch Extraktion mit Diethylether und anschließender Aufkonzentrierung gewonnen. Die Identifizierung erfolgte durch GC/MS.

### Beispiel 2: Herstellung von 2-substituierten N-(2-Mercaptoethyl)-1,3-thiazolidinen

### a) Herstellung der 2-substituierten Thiazolidine

1,1 mmol Carbonyl- oder Dicarbonylverbindung werden mit 1,1 mmol Cysteaminhydrochlorid in sauerstofffreier, wässriger Phosphatpufferlösung (pH=7) unter Schutzgasatmosphäre umgesetzt. Die Umsetzung erfolgt bei den reaktiven Komponenten Formaldehyd, Acetaldehyd und Methylglyoxal bei 0°C. Die Umsetzung wird gaschromatographisch verfolgt. Nach Aufarbeitung und chromatographischer Reinigung erhält man die entsprechenden Thiazolidine.

### b) Herstellung der 2-substituierten N-(2-Mercaptoethyl)- 1 ,3-thiazolidinen

10 mmol 2-substituiertes Thiazolidin werden in einem Glasautoklaven mit 10 mmol Ethylensulfid versetzt und ohne Lösungsmittel unter Schutzgas 24 Stunden auf 80°C erhitzt. Das Reaktionsgemisch wird in Natronlauge aufgenommen und mit Dichlormethan gewaschen. Die wässrige Phase wird mit Salzsäure auf pH 8 eingestellt und mit Dichlormethan extrahiert. Man erhält die N-(2-Mercaptoethyl)-thiazolidine in einer Reinheit von ca. 90-96% und in einer Ausbeute von 75-88%.

Massenspektrum von N-(2-Mercaptoethyl)-thiazolidin

| m/z | Intensität % |
|---|---|
| 27 | 12 |
| 28 | 12 |
| 42 | 45 |
| 45 | 12 |
| 56 | 12 |
| 57 | 13 |
| 59 | 9 |
| 61 | 15 |
| 88 | 7 |
| 102 | 100 |

### Beispiel 3: Herstellung eines Röst-Aromas

Es wurden vermischt (alle Angaben in g):

| | |
|---|---|
| 3-Methylthiopropanal (1%ig in Pflanzenöltriglyceriden) | 1,0 |
| 2,3-Diethyl-5-methylpyrazin | 1,0 |
| Isoamylcaprylat | 1,0 |
| Diacetyl (10%ig in Triacetin) | 2,0 |
| 2-Methylbuttersäure | 5,0 |
| Isoamylalkohol | 10,0 |
| Delta-Dodecalacton | 10,0 |
| 2-Phenylethanol | 15,0 |
| 2-Methylbutanal | 20,0 |
| Caprylsäure (10%ig in Triacetin) | 25,0 |
| Dimethyloxyfuron (1%ig in Propylenglycol) | 100,0 |
| 2,5-Dimethyl-4-hydroxy-3(2H)-furanon (15%ig in Propylenglycol) | 500,0 |
| Pflanzenöltriglyceride | 9310,0 |
| Summe | 10000,0 |

Ersetzte man 0,1 - 0,5 g des Lösungsmittels Pflanzenöltriglyceride durch 0,1 - 0,5 g N-(2-Mercaptoethyl)-thiazolidin, so wurde das Aroma deutlich typischer in Richtung röstig, Brotkruste.

## Patentansprüche

1. Verbindungen der Formel 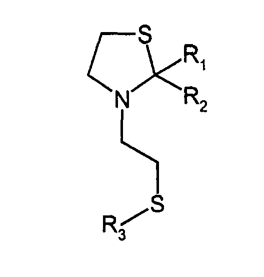 in denen R_{1,} R₂ und R₃ gleich oder verschieden sind und R₁ Wasserstoff, Methyl, Ethyl, Propyl, iso Propyl bedeutet, R₂ Wasserstoff, Acetyl, Propionyl, Isobutyryl, Methyl, Ethyl, Propyl, iso Propyl und R₃ Wasserstoff oder 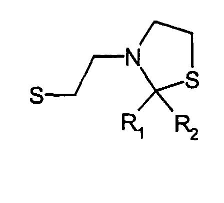 bedeutet,
mit der Maßgabe, dass es sich nicht um 2,2-Dimethyl-3-thiazolidinethanthiol handelt.

2. Verbindung nach Anspruch 1, wobei es sich um N-(2-Mercaptoethyl)-1,3-thiazolidin handelt.

3. Riech- und Aromastoffkompositionen enthaltend Verbindungen der Formel 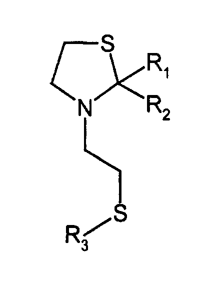 in denen R₁, R₂ und R₃ gleich oder verschieden sind und R₁ Wasserstoff, Methyl, Ethyl, Propyl, iso Propyl bedeutet, R₂ Wasserstoff, Acetyl, Propionyl, Isobutyryl, Methyl, Ethyl, Propyl, iso Propyl und R₃ Wasserstoff oder 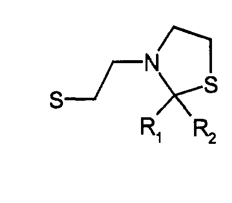 bedeutet.

4. Riech- und Aromastoffkompositionen enthaltend die Verbindung nach Anspruch 2.

5. Verwendung von N-(2-Mercaptoethyl)-1,3-thiazolidin als Aromastoff in flüssigen und trockenen Aromen für Nahrungs- und Genussmittel, zum Versprühen von Lebensmitteldüften und zur Raumluftaromatisierung.

6. Verwendung von N-(2-Mercaptoethyl)-1,3-thiazolidin zur Aromatisierung von Verpackungen von Lebens- und Genussmitteln.

## Claims

1. Compounds of the formula 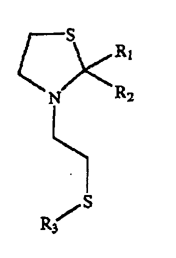 in which R₁, R₂ and R₃ are identical or different and R₁ means hydrogen, methyl, ethyl, propyl or isopropyl, R₂ means hydrogen, acetyl, propionyl, isobutyryl, methyl, ethyl, propyl or isopropyl and R₃ means hydrogen or 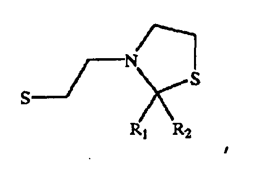 provided that they are not 2,2-dimethyl-3-thiazolidineethanethiol.

2. A compound according to claim 1, wherein said compound is N-(2-mercaptoethyl ) -1, 3-thiazolidine.

3. Fragrance and flavouring compositions containing compounds of the formula 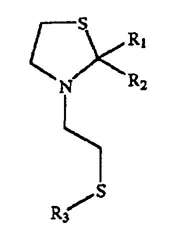 in which R₁, R₂ and R₃ are identical or different and R₁ means hydrogen, methyl, ethyl, propyl or isopropyl, R₂ means hydrogen, acetyl, propionyl, isobutyryl, methyl, ethyl, propyl or isopropyl and R₃ means hydrogen or

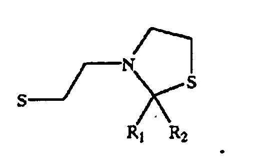

4. Fragrance and flavouring compositions containing the compound according to claim 2.

5. Use of N-(2-mercaptoethyl)-1,3-thiazolidine as a flavouring in liquid and dry flavours for foods and semi-luxury products, for spraying food aromas and for indoor.air fragrancing.

6. Use of N-(2-mercaptoethyl)-1,3-thiazolidine for fragrancing food and semi-luxury product packaging.

## Revendications

1. Composés de formule 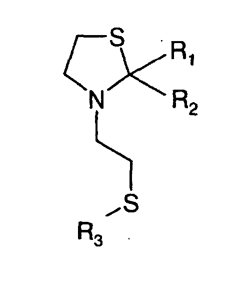 où R₁, R₂ et R₃ sont identiques ou différents et R₁ représente l'hydrogène, méthyle, éthyle, propyle, isopropyle, R₂ représente l'hydrogène, acétyle, propionyle, isobutyryle, méthyle, éthyle, propyle, isopropyle et R₃ représente l'hydrogène ou 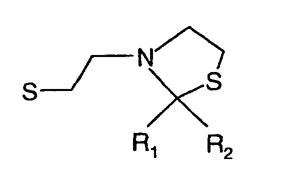 à condition qu'il ne s'agisse pas du 2,2-diméthyl-3-thiazolidlneéthanethiol.

2. Composé selon la revendication 1 où il s'agit de la N-(2-mercaptoéthyl)-1,3-thiazolidine.

3. Compositions de substances odorantes et aromatisantes contenant des composés de formule 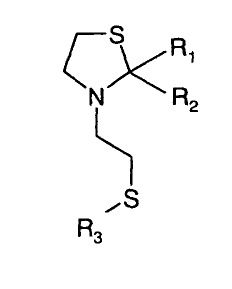 où R₁, R₂ et R₃ sont identiques ou différents et R₁ représente l'hydrogène, méthyle, éthyle, propyle, isopropyle, R₂ représente l'hydrogène, acétyle, propionyle, isobutyryle, méthyle, éthyle, propyle, isopropyle et R₃ représente l'hydrogène ou

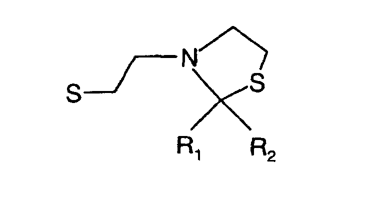

4. Compositions de substances odorantes et aromatisantes contenant le composé selon la revendication 2.

5. Utilisation de la N-(2-mercaptoéthyl)-1,3-thiazolidine comme substance aromatisante dans des arômes liquides et secs pour produits alimentaires et de consommation, pour la pulvérisation de parfums alimentaires et pour l'aromatisation de l'air ambiant.

6. Utilisation de la N-(2-mercaptoéthyl)-1,3-thiazolidine pour l'aromatisation d'emballages de produits alimentaires et de consommation.
